(19) Europäisches Patentamt
European Patent Office
Office européen des brevets

(11) **EP 4 729 931 A1**

(12) **EUROPEAN PATENT APPLICATION**
published in accordance with Art. 153(4) EPC

(43) Date of publication:
**22.04.2026 Bulletin 2026/17**

(21) Application number: **24879375.4**

(22) Date of filing: **24.06.2024**

(51) International Patent Classification (IPC):
***G01N 21/89*** (2006.01)

(52) Cooperative Patent Classification (CPC):
**G01N 21/89**

(86) International application number:
**PCT/JP2024/022888**

(87) International publication number:
**WO 2025/083944 (24.04.2025 Gazette 2025/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **17.10.2023 JP 2023178784**

(71) Applicant: **Hamamatsu Photonics K.K.
Hamamatsu-shi, Shizuoka 435-8558 (JP)**

(72) Inventors:
• **TSUCHIYA, Kunihiko**
  **Hamamatsu-shi, Shizuoka 435-8558 (JP)**
• **OHTSUKA, Kenichi**
  **Hamamatsu-shi, Shizuoka 435-8558 (JP)**
• **MATSUDA, Shunsuke**
  **Hamamatsu-shi, Shizuoka 435-8558 (JP)**
• **KONDO, Hideyuki**
  **Hamamatsu-shi, Shizuoka 435-8558 (JP)**

(74) Representative: **Grünecker Patent- und
Rechtsanwälte
PartG mbB
Leopoldstraße 4
80802 München (DE)**

(54) **INSPECTION DEVICE**

(57) An inspection device 1 for detecting foreign matter F, which is a transparent substance, in an object S comprises: an irradiation unit 3 configured to irradiate the object S with inspection light L0 including a plurality of wavelengths; a spectroscopic detection unit 4 configured to detect light L1 from an irradiation position R of the inspection light L0 in the object S and output a spectroscopic spectrum D indicating luminance of each wavelength at the irradiation position R; and a determination unit 5 configured to determine presence or absence of the foreign matter F at the irradiation position R based on information about periodicity of the spectroscopic spectrum D.

*Fig.1*

EP 4 729 931 A1

## Description

## Technical Field

The present disclosure relates to an inspection device.

Background Art

Conventionally, an inspection device for inspecting the presence or absence of foreign matter in an object and the like by irradiating the object with light is known. An example of this type of inspection device include the inspection device described in Patent Literature 1. In this conventional inspection device, transmitted light transmitted through an object and reflected light reflected by the object are imaged by an imaging unit and the object is inspected based on detection data output from the imaging unit.

## Citation List

## Patent Literature

[Patent Literature 1] Japanese Unexamined Patent Publication No. 2021-189071

## Summary of Invention

## Technical Problem

Although various types of foreign matter may be located in an object, the foreign matter is also assumed to include transparent substances whose shapes are difficult to identify, such as plastic fragments. When the foreign matter is a transparent substance, it may be difficult to detect the presence or absence of the foreign matter simply by observing transmitted light transmitted through the object and reflected light reflected by the object.

The present disclosure has been made to solve the above-described problems and an objective of the present disclosure is to provide an inspection device that can accurately detect the presence or absence of foreign matter, which is a transparent substance.

## Solution to Problem

The subject matter of the present disclosure is as shown in the following [1] to [9].

[1] There is provided an inspection device configured to detect foreign matter, which is a transparent substance, in an object, the inspection device comprising: an irradiation unit configured to irradiate the object with inspection light including a plurality of wavelengths; a spectroscopic detection unit configured to detect light from an irradiation position of the inspection light on the object and output a spectroscopic spectrum indicating luminance of each wavelength at the irradiation position; and a determination unit configured to determine presence or absence of the foreign matter at the irradiation position based on information regarding periodicity of the spectroscopic spectrum.

This inspection device determines the presence or absence of the foreign matter at the irradiation position on the object based on the information about the periodicity of the spectroscopic spectrum indicating the luminance of each wavelength at the irradiation position of the inspection light. When the foreign matter is the transparent substance, the inspection light is incident on the foreign matter, such that specularly reflected light specularly reflected from the surface of the foreign matter, specularly reflected light transmitted through the foreign matter and specularly reflected from the back surface, and multiple-reflected light in which light is transmitted through the foreign matter and multiple-reflected within the foreign matter can be generated. Because the reflected light beams intensify or weaken each other in accordance with a phase difference determined by a wavelength and an optical path length, if the detected reflected light is spectrally separated, the spectroscopic spectrum having the periodicity of the peaks and valleys on the wavelength axis is obtained. Therefore, by using the inspection light containing a plurality of wavelengths to spectrally separate the reflected light and acquiring information about the periodicity of the spectroscopic spectrum, it is possible to accurately detect the presence or absence of the foreign matter, which is the transparent substance.

[2] In the inspection device according to [1], the determination unit configured to perform a Fourier transform on the spectroscopic spectrum and determine the presence or absence of the foreign matter at the irradiation position by comparing a peak value of an intensity after the Fourier transform with a predetermined threshold. By performing the Fourier transform on the spectroscopic spectrum, it is possible to easily ascertain whether or not there is a periodic interference component in the spectroscopic spectrum based on whether or not a peak appears in the intensity after the Fourier transform. Thereby, it is possible to perform more accurate detection of the presence or absence of the foreign matter, which is the transparent substance.

[3] In the inspection device according to [2], the determination unit configured to accumulate the luminance of the spectroscopic spectrum in a predetermined wavelength range and perform the Fourier transform on the spectroscopic spectrum when an accumulated value exceeds a predetermined threshold. When the foreign matter, which is the transparent substance, is present, because the specularly reflected light from the foreign matter is detected, the luminance of the detected light tends to be higher than that of an area where there is no foreign matter. Therefore, by performing the Fourier transform on the spectroscopic spectrum only when the accumulated value of the luminance of the spectroscopic spectrum in a predetermined wavelength range exceeds a predetermined threshold, it is possible to reduce the processing load in the determination unit until the presence or absence of the foreign matter is determined.

[4] In the inspection device according to any one of [1] to [3], a wavelength range included in the inspection light is greater than or equal to 1000 nm and less than 2500 nm. As described above, the phase difference of reflected light is determined by the wavelength of light and the optical path length. Therefore, if the thickness of the foreign matter, which is the transparent substance, increases, it is considered that the periodicity of the spectroscopic spectrum is short and the accuracy of determining the presence or absence of the foreign matter decreases. On the other hand, by setting the wavelength contained in the detection light to a long wavelength as in the above-described range, the periodicity of the spectroscopic spectrum is extended even if the thickness of the foreign matter is large, and the accuracy of determining the presence or absence of the foreign matter can be sufficiently guaranteed.

[5] In the inspection device according to any one of [1] to [4], the irradiation unit includes a line illuminator. In this case, a plurality of irradiation positions can be formed for the object at once. Therefore, it is possible to quickly inspect the foreign matter of the object.

[6] In the inspection device according to any one of [1] to [5], the irradiation unit includes a dome illuminator or a flat dome illuminator. If the surface of the object is uneven or sloping, it is considered that the detection efficiency of specularly reflected light from foreign matter may be reduced. When the dome illuminator or flat dome illuminator is used, it is possible to cover the angle range corresponding to the unevenness and slope of the surface of the object, and the accuracy of determining the presence or absence of the foreign matter can be sufficiently guaranteed even if there is unevenness or a slope on the surface of the object.

[7] In the inspection device according to any one of [1] to [6], the spectroscopic detection unit includes a multi-wavelength spectral camera. By using a multi-wavelength spectral camera as the spectroscopic detection unit, for example, it is possible to acquire a linear spectroscopic spectrum with a resolution of 100 wavelengths or more. Therefore, the periodicity of the spectroscopic spectrum can be acquired in a sufficient wavelength range, and the presence or absence of the foreign matter can be suitably determined.

[8] In the inspection device according to any one of [1] to [4], the irradiation unit includes a line illuminator, the spectroscopic detection unit includes a point spectroscopic detector, and the inspection device has a scanner configured to scan a detection field of view of the spectroscopic detection unit. In this case, the spectroscopic detection unit can be configured using an existing spectroscopic detector.

[9] In the inspection device according to any one of [1] to [4], the irradiation unit includes point illumination, the spectroscopic detection unit includes a point spectroscopic detector, and the inspection device has a scanner configured to scan an irradiation position of the irradiation unit and a detection field of view of the spectroscopic detection unit. In this case, the spectroscopic detection unit can be configured using an existing spectroscopic detector.

[10] In the inspection device according to any one of [1] to [9], a polarizer in a parallel Nicol relationship is provided in each of the irradiation unit and the spectroscopic detection unit. In the specularly reflected light and multiple-reflected light from the object, the polarized state of the inspection light radiated onto the object is maintained, while the diffused light on the surface of the object is non-polarized. Therefore, by providing a polarizer with a parallel Nicol relationship between the irradiation unit and the spectroscopic detection unit, it is possible to eliminate background influences caused by diffusely reflected light or the like and to detect the presence or absence of the foreign matter, which is the transparent substance, with higher accuracy.

[11] The inspection device according to any of [1] to [10] further comprises a transport unit configured to transport the object in a predetermined direction. In this case, because the irradiation area of the inspection light can be scanned for the object, it is possible to quickly and easily determine the presence or absence of the foreign matter over a wide range of the object.

**Advantageous Effects of Invention**

According to the present disclosure, it is possible to accurately detect the presence or absence of foreign matter, which is a transparent substance.

**Brief Description of Drawings**

> FIG. 1 is a schematic diagram showing a configuration of an inspection device according to an embodiment of the present disclosure.
> FIGS. 2(a) to 2(c) are diagrams showing an overview of the inspection of foreign matter of an object by the inspection device shown in FIG. 1.

FIG. 3 is a schematic diagram showing a state of the reflection of inspection light on the object.

FIG. 4 is a schematic diagram showing the enlargement of a state of the reflection of the inspection light on the foreign matter.

FIGS. 5(a) to 5(c) are schematic diagrams showing a process for analyzing a spectroscopic spectrum in a determination unit.

FIGS. 6(a) to 6(c) are diagrams showing changes in the spectroscopic spectrum acquired in a near-infrared wavelength range relative to a thickness of the foreign matter.

FIGS. 7(a) to 7(c) are diagrams showing changes in the spectroscopic spectrum acquired in the wavelength range of shortwave infrared light relative to a thickness of foreign matter.

FIG. 8 is a flowchart showing an example of a foreign matter inspection method using the inspection device shown in FIG. 1.

FIG. 9 is a schematic diagram showing imaging conditions of specularly reflected light from foreign matter when there is an unevenness or slope on a surface of foreign matter.

FIG. 10 is a schematic diagram showing an example of a dome illuminator.

FIG. 11 is a schematic diagram showing an example of a flat dome illuminator.

FIG. 12 is a schematic diagram showing a modification example of an irradiation unit and a spectroscopic detection unit.

FIG. 13 is a schematic diagram showing another modification example of the irradiation unit and the spectroscopic detection unit.

FIG. 14 is a schematic diagram showing yet another modification example of the irradiation unit and the spectroscopic detection unit.

**Description of Embodiments**

Hereinafter, a preferred embodiment of an inspection device according to an aspect of the present disclosure will be described in detail with reference to the drawings.

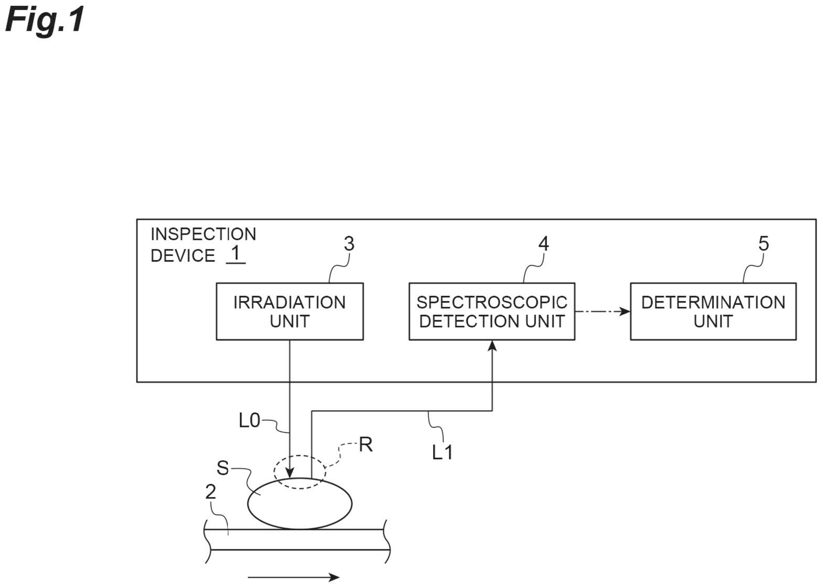

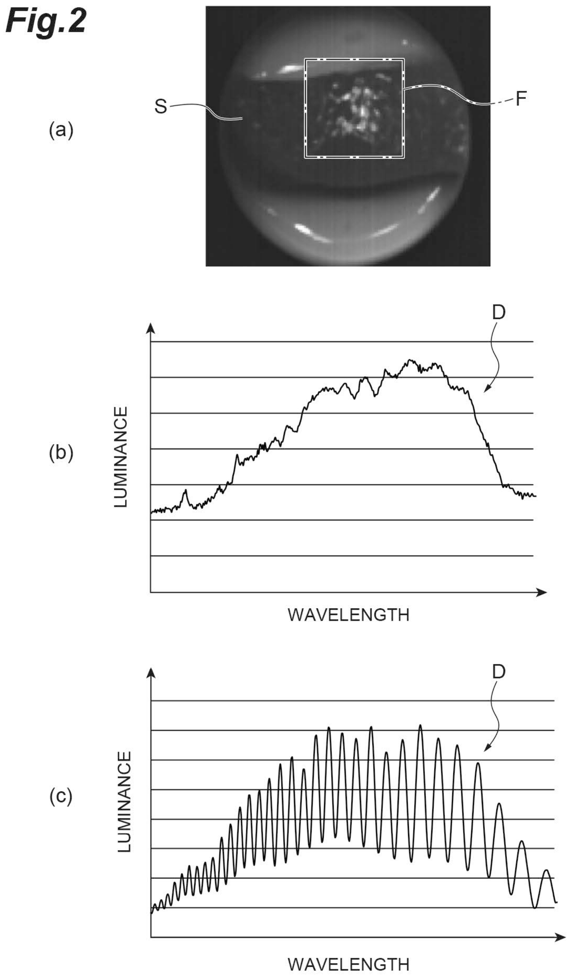

FIG. 1 is a schematic diagram showing a configuration of an inspection device according to an embodiment of the present disclosure. The inspection device 1 shown in FIG. 1 is configured as a device that inspects foreign matter F in an object S by irradiation of inspection light L0. In the inspection device 1, the inspection light L0 containing a plurality of wavelengths is radiated to the object S, and a spectroscopic spectrum D indicating the luminance of each wavelength is acquired by detecting light L1 from the object S at an irradiation position R of the inspection light L0 (see FIGS. 2(a) and 2(b)). Also, based on information about the periodicity of the spectroscopic spectrum D, the presence or absence of the foreign matter F at the irradiation position R of the object S is determined.

Examples of the object S include foods. Examples of the foods include, for example, beef, pork, chicken, lamb, or processed foods thereof. The object S is not limited to foods, and may also be other goods such as electronic parts. The foreign matter F includes transparent substances such as plastic fragments here. The object S may be either transparent or non-transparent to the inspection light L0. The object S transparent to the inspection light L0 includes, for example, a liquid such as water, and in this case, the foreign matter F is an oil film or the like. Moreover, as the object S transparent to the inspection light L0 includes glass, and in this case, the foreign matter F is a film or the like.

FIGS. 2(a) to 2(c) are diagrams showing an overview of the inspection of the foreign matter of the object S by the inspection device 1. In FIG. 2(a), an example of chicken is shown as the object S, and a state in which a transparent plastic fragment adhered to a surface of chicken as the foreign matter F is shown. Although periodicity is not observed in the acquired spectroscopic spectrum D in an area where there is no plastic fragment on the surface of the chicken as shown in FIG. 2(b), periodicity due to spectroscopic interference is observed in the acquired spectroscopic spectrum D in an area where there is a plastic fragment on the surface of the chicken as shown in FIG. 2(c). Therefore, by analyzing information about the periodicity of the spectroscopic spectrum D, it is possible to determine the presence or absence of the foreign matter F at the irradiation position R of the object S.

Hereinafter, a specific configuration of the inspection device 1 will be described. As shown in FIG. 1, in the present embodiment, the inspection device 1 is configured to include a transport unit 2, an irradiation unit 3, a spectroscopic detection unit 4, and a determination unit 5. The transport unit 2 is a part that transports the object S in a predetermined direction. The transport unit 2, for example, includes a conveyor belt. The transport unit 2 transports the object S horizontally at a certain speed V toward the irradiation position R of the inspection light L0 by the irradiation unit 3. Thereby, the irradiation position R is scanned with respect to the object S.

The irradiation unit 3 is a part that irradiates the object S with the inspection light L0 containing a plurality of wavelengths. The irradiation unit 3 has a light-emitting device and an illumination circuit for turning on the light-emitting device, and diffuses the inspection light L0 toward the object S and radiates the inspection light L0. The light-emitting device constituting the irradiation unit 3 includes, for example, LEDs, super luminescent diodes (SLDs), lasers, halogen lamps, fluorescent lamps, organic EL, and the like.

In the present embodiment, the irradiation unit 3 includes a line illuminator 11 in which a light-emitting device is arranged in a direction perpendicular to the transport direction of the object S in the transport unit 2. By using the line illuminator 11, the irradiation position R of the inspection light L0 is formed on the surface of the object S in a line shape. A wavelength range of the inspection light L0 includes, for example, a near-infrared range (greater than or equal to 750 nm to less than 1000 nm), a shortwave infrared range (greater than or equal to 1000 nm to less than 2500 nm), or the like. Among these wavelength ranges, it is preferable to select the shortwave infrared range from the perspective of ensuring the detection accuracy of foreign matter F with a large thickness (details will be described below).

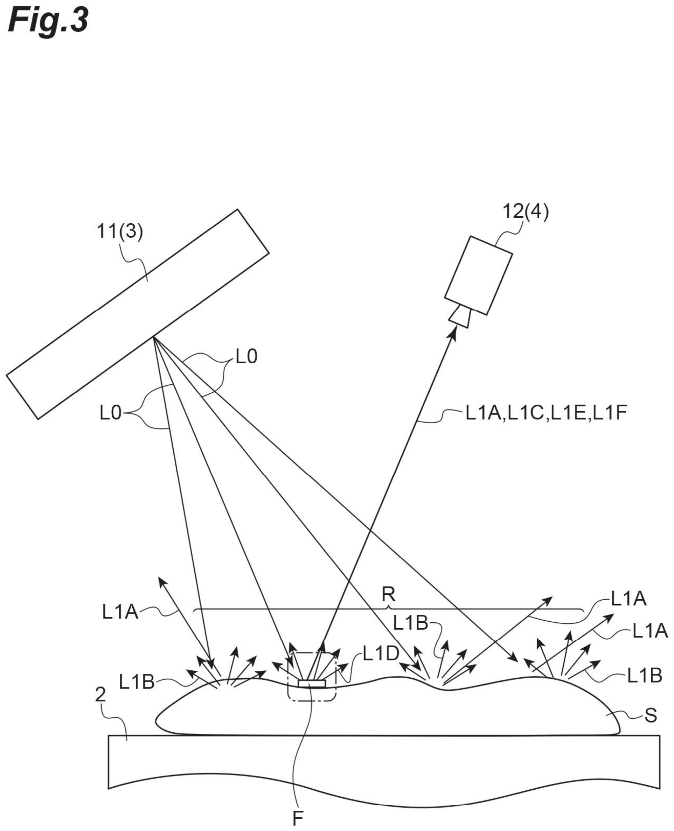

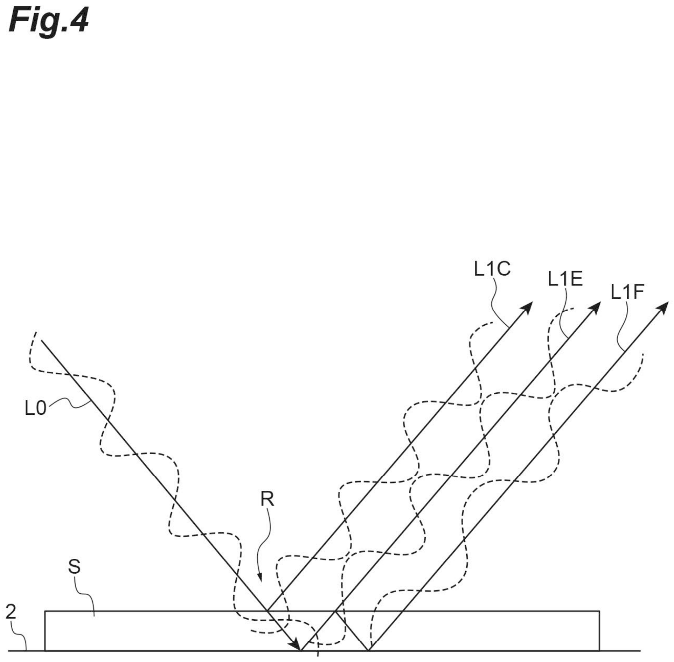

FIG. 3 is a schematic diagram showing a state of the reflection of inspection light on the object. Moreover, FIG. 4 is a schematic diagram showing the enlargement of a state of the reflection of the inspection light on the foreign matter. As shown in FIGS. 3 and 4, the inspection light L0 emitted from the irradiation unit 3 is radiated to the surface of the object S in a widely diffused state. On the surface of the object S irradiated with the inspection light L0, specularly reflected light L1A and diffusely reflected light L1B can be generated. If the foreign matter F, which is the transparent substance, is located on the surface of the object S, specularly reflected light L1C and diffusely reflected light L1D can be generated on the surface of the foreign matter F irradiated with the inspection light L0. Moreover, there may be specularly reflected light L1E transmitted through the foreign matter F and specularly reflected on the back side and multiple-reflected light L1F in which light transmitted through the foreign matter F is multiple-reflected within the foreign matter F. When the foreign matter F is the transparent substance and the object S is a non-transparent substance, intensities of specularly reflected light L1C and L1E tend to be higher than those of the specularly reflected light L1A and the diffusely reflected light L1B.

The spectroscopic detection unit 4 detects the light L1 from the irradiation position R of the inspection light L0 in the object S, and outputs the spectroscopic spectrum D indicating the luminance of each wavelength at the irradiation position R. The spectroscopic detection unit 4 detects the light L1 from the irradiation position R through an imaging lens (not shown), as shown in FIG. 3. The light L1 from the irradiation position R serving as a detection target of the spectroscopic detection unit 4 is mainly the specularly reflected light L1A from the object S when there is no foreign matter F, and is mainly the specularly reflected light L1C, the specularly reflected light L1E, and the multiple-reflected light L1F from the foreign matter F when there is the foreign matter F. The detection axis of the spectroscopic detection unit 4 is arranged symmetrically with the optical axis of the inspection light L0 with respect to the normal line of the object S (the normal line of the placement surface of the object S in the transport unit 2) so that the specularly reflected light L1A, the specularly reflected light L1C, the specularly reflected light L1E, and the multiple-reflected light L1F are efficiently incident.

The spectroscopic detection unit 4 includes, for example, a linear spectroscopic detector 12. The linear spectroscopic detector 12 outputs the spectroscopic spectrum D at each position of the line-shaped irradiation position R based on the light L1 from the line-shaped irradiation position R from the object S. As the linear spectroscopic detector 12, for example, a multi-wavelength spectral camera such as a multispectral camera or a hyperspectral camera can be used. The multi-wavelength spectral camera is, for example, a camera that can acquire a spectroscopic spectrum D with a resolution of 10 wavelengths or more. The multispectral camera, which is a type of multi-wavelength spectral camera, is a camera into which a two-dimensional image sensor and a wavelength filter are built, and, for example, can output the spectroscopic spectrum D with a resolution of 10 wavelengths or more. A hyperspectral camera, which is a type of multi-wavelength spectral camera, is a camera into which a two-dimensional image sensor, slit, and grating are built, and, for example, can output the spectroscopic spectrum D with a resolution of 100 wavelengths or more. Although linear spectral data of two axes of the wavelength axis and the spatial axis is output from the linear spectroscopic detector 12, a hyperspectral camera with a higher wavelength resolution is adopted in the present embodiment.

The determination unit 5 is a part that determines the presence or absence of the foreign matter F at the irradiation position R based on information about the periodicity of the spectroscopic spectrum D. The determination unit 5 is a computer that physically includes storage devices such as a RAM and a ROM, a processor (computing circuit) such as a CPU, a communication interface, and the like. As a computer, for example, it is possible to use a personal computer, a cloud server, or a smart device (a smartphone, a tablet device, or the like). The determination unit 5 may include a field programmable gate array (FPGA), an application specific integrated circuit (ASIC), or the like. The determination unit 5 is connected to the spectroscopic detection unit 4 by a wired or wireless link so that information communication is possible.

In the present embodiment, when the presence or absence of the foreign matter F based on the spectroscopic spectrum D is determined, the determination unit 5 converts the spectroscopic spectrum D indicating the luminance per wavelength received from the spectroscopic detection unit 4 into the spectroscopic spectrum D indicating an amplitude per wavenumber (a reciprocal of the wavelength). Also, the Fourier transform is performed on the spectroscopic spectrum D for each wavenumber, and the presence or absence of foreign matter at the irradiation position R is determined by comparing a peak value of the intensity after the Fourier transform with a predetermined threshold.

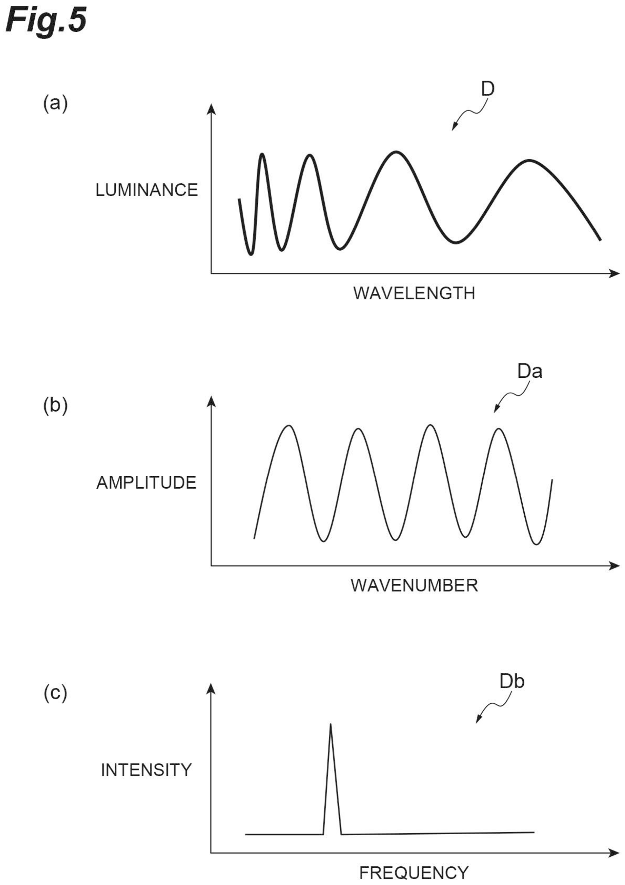

FIGS. 5(a) to 5(c) are schematic diagrams showing a process for analyzing a spectroscopic spectrum D in determination unit 5. As shown in FIG. 5(a), the spectroscopic spectrum D output from the spectroscopic detection unit 4 is expressed as the luminance relative to the wavelength. When there is no foreign matter F in the object S, the spectroscopic detection unit 4 mainly detects the specularly reflected light L1A from the object S. In this case, a spectroscopic spectrum D that does not have periodicity is obtained, as shown in FIG. 2(b).

On the other hand, when the foreign matter F, which is the transparent substance, is located in the object S, it is possible to generate the specularly reflected light L1C specularly reflected on the surface of the foreign matter F, the specularly reflected light L1E transmitted through the foreign matter F and specularly reflected on the back side, and the multiple-reflected light L1F in which the light transmitted through the foreign matter F is multiple-reflected within the foreign matter F as shown in FIG. 4. Because the reflected light beams intensify or weaken each other depending on a phase difference determined by the wavelength and the optical path length, if the detected reflected light is spectrally separated, the spectroscopic spectrum D having the periodicity of the peaks and valleys on the wavelength axis is obtained, as shown in FIG. 5(a).

When the foreign matter F is the transparent substance, the spectroscopic spectrum D detected by the spectroscopic detection unit 4 is expressed by the following formula (1). In formula (1), I denotes the luminance (intensity) of the reflected light, $\lambda$ denotes a wavelength of the inspection light, n denotes a refractive index of the foreign matter, d denotes a thickness of the foreign matter, and A and B denote constants determined by the reflectance of the foreign matter's interface.

[Math. 1]

$$I = A \times cos\left(2\pi \frac{2nd}{\lambda}\right) + B \cdots (1)$$

When the spectroscopic spectrum D indicating the luminance per wavelength shown in FIG. 5(a) is converted into an amplitude per wavenumber, a spectroscopic spectrum Da with periodicity of a certain period is obtained, as shown in FIG. 5(b). If the Fourier transform is performed on this spectroscopic spectrum Da, a spectroscopic spectrum Db indicating an intensity relative to a frequency is obtained as shown in FIG. 5(c). A frequency indicating a peak value of an intensity of the spectroscopic spectrum Db corresponds to a thickness of the foreign matter F. The determination unit 5 detects the peak value of the intensity of the spectroscopic spectrum Db after the Fourier transform, determines that there is no foreign matter F in the object S when the peak value is less than or equal to a predetemined threshold, and determines that there is foreign matter F in the object when the peak value exceeds the predetermined threshold.

A relationship between the spectroscopic spectrum D shown in FIG. 5(a) and the thickness of the foreign matter F is expressed in the following formula (2). In formula (2), n denotes a refractive index of the foreign matter, d denotes a thickness of the foreign matter, $\varphi$ denotes an angle of incidence of the inspection light, m denotes the number of peaks and valleys in the calculated wavelength range, and $\lambda$1 and $\lambda$2 denote wavelengths of the start and end points of the calculated wavelength range.

[Math. 2]

$$d = \frac{m}{2\sqrt{n^2 + sin^2 \varphi}} \times \frac{1}{\left(\frac{1}{\lambda_2} - \frac{1}{\lambda_1}\right)} \cdots (2)$$

By the modification of the above formula (2), the number of peaks and valleys in the calculated wavelength range is expressed by the following formula (3). From formula (3), it can be seen that the number of peaks and valleys in the calculated wavelength range increases as the thickness of the foreign matter F increases. Because the period of the peaks and valleys in the calculated wavelength range decreases if the number of peaks and valleys in the calculated wavelength range increases, the detection accuracy of the foreign matter F may decrease if the wavelength resolution of the spectroscopic detection unit 4 is insufficient.

[Math. 3]

$$= 2d\sqrt{n^2 + sin^2 \varphi} \times \frac{(\lambda_1 - \lambda_2)}{\lambda_1 \times \lambda_2} \cdots (3)$$

On the other hand, it can be seen that the longer the wavelength used in the calculated wavelength range from formula (3), the fewer the number of peaks and valleys in the calculated wavelength range and the longer the period of peaks and valleys in the calculated wavelength range. Therefore, by selecting a plurality of wavelengths contained in the inspection light L0 so that the period of the valley in the calculated wavelength range is longer than the wavelength resolution of the spectroscopic detection unit 4, the detection accuracy of the foreign matter F can be ensured even when the thickness of the foreign matter F is large.

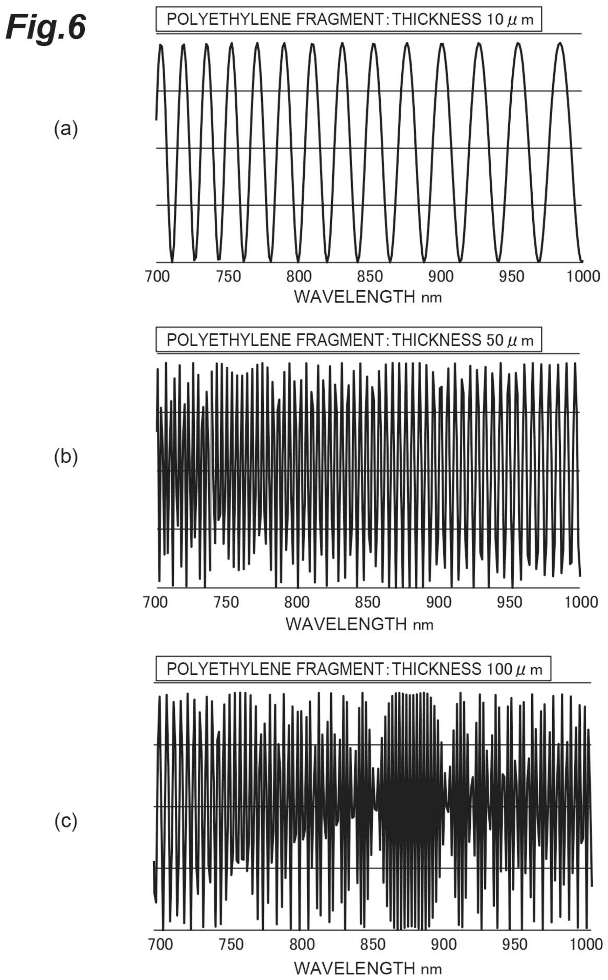

FIGS. 6(a) to 6(c) are diagrams showing states of changes in the thickness of the foreign matter in the spectroscopic spectrum acquired in the wavelength range of the near-infrared light (a wavelength greater than or equal to 700 nm and less than 1000 nm). Here, a polyethylene fragment was used as the foreign matter, which is the transparent substance. In FIGS. 6(a) to 6(c), states of the spectroscopic spectra of polyethylene fragments with thicknesses of 10 $\mu$m, 50 $\mu$m, and 100 $\mu$m are shown. From these results, it can be confirmed that, when the wavelength range of the inspection light is kept constant, the period of the peaks and valleys actually decreases in the calculated wavelength range as the thickness of the transparent substance increases.

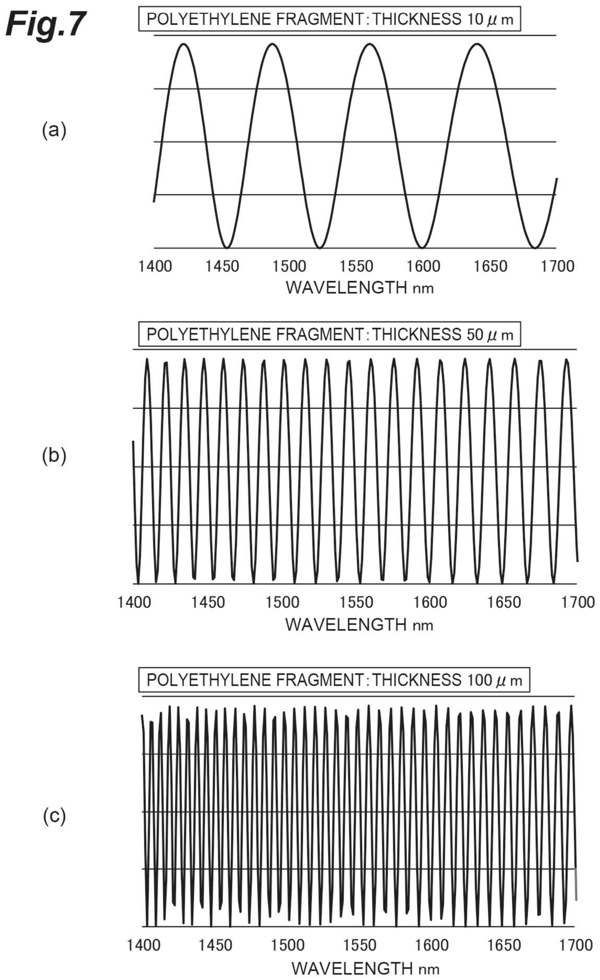

On the other hand, FIGS. 7(a) to 7(c) are diagrams showing states of changes in the spectroscopic spectra acquired in the wavelength range of shortwave infrared light (a wavelength greater than or equal to 1000 nm and less than 2500 nm) relative to the thickness of the foreign matter. In FIGS. 7(a) to 7(c), as in the cases of FIGS. 6(a) to 6(c), the states of the spectroscopic spectra when a polyethylene fragment is used as the foreign matter, which is the transparent substance, and the thickness of the polyethylene fragment is 10 $\mu$m, 50 $\mu$m, and 100 $\mu$m respectively, are shown. From these results, it can be confirmed that the period of the peaks and valleys in the calculated wavelength range actually increases as the wavelength range of the inspection light increases for the thickness of the same transparent substance.

Therefore, by setting the wavelength range of the inspection light L0 to shortwave infrared light (a wavelength greater than or equal to 1000 nm and less than 2500 nm), the detection accuracy of the foreign matter F can be ensured even if the thickness of foreign matter F is large. For example, when a polyethylene fragment with a thickness of 100 $\mu$m is the foreign matter, if the calculated wavelength range is 1600 $\mu$m to 1700 $\mu$m, a valley in the spectroscopic spectrum will appear for about 12 periods in the range. In this case, if the spectroscopic detection unit 4 has a wavelength resolution of approximately 8 $\mu$m or less, the detection accuracy of foreign matter F can be sufficiently guaranteed.

Moreover, in the present embodiment, from the viewpoint of reducing the processing load in the determination unit 5, the determination unit 5 accumulates the luminance of the spectroscopic spectrum D in a predetermined wavelength range, and the Fourier transform is performed on the spectroscopic spectrum D when the accumulated value exceeds the predetermined threshold. As shown in FIG. 3, irradiation of the inspection light L0 can produce light containing the specularly reflected light L1C in the foreign matter F, which is the transparent substance. When the object S is a non-transparent substance, the luminance of the specularly reflected light L1C from the foreign matter F, which is the transparent substance, tends to be higher than the luminance of the specularly reflected light L1A from the object S. Therefore, the processing load in the determination unit 5 is reduced by performing the Fourier transform on the spectroscopic spectrum D only when the accumulated value of the luminance of the spectroscopic spectrum D in a predetermined wavelength range exceeds the predetermined threshold. In addition, the predetermined wavelength range for accumulating the luminance of spectroscopic spectrum D may be, for example, a detection wavelength range of a multi-wavelength spectral camera or a wavelength range, which is a part of the detection wavelength range.

When there is an unevenness or slope on the surface of the object S, it is considered that the inspection light L0 does not hit the surface of the object S and a shadow is formed. In the shadow part, the noise in the spectroscopic detection unit 4 is relatively large, and the detection accuracy of foreign matter F may decrease as a result of the increase in the amplitude of the spectroscopic spectrum D due to the noise. For such phenomena, the Fourier transform can be performed on the spectroscopic spectrum D only when the accumulated value of the luminance of spectroscopic spectrum D in a predetermined wavelength range exceeds a predetermined threshold, such that the amplitude of the spectroscopic spectrum D of noise can be separated from the amplitude of the spectroscopic spectrum D of the specularly reflected light L1C from the foreign matter F, and the accuracy of detection of the foreign matter F can be sufficiently guaranteed.

Moreover, when the object S is a transparent substance such as a mirror surface or a liquid, it is considered that the luminance of the specularly reflected light L1A from the object S can be higher than or equal to that of the specularly reflected light L1C from the foreign matter F. In this case, it is difficult to separate the amplitude of the spectroscopic spectrum D of the specularly reflected light L1C from the foreign matter F and the amplitude of the spectroscopic spectrum D of the specularly reflected light L1A from the object S by the accumulated value of the luminance of the spectroscopic spectrum D in a predetermined wavelength range, but there is no influence on the determination of the presence or absence of the foreign matter F based on the peak value of the intensity of the spectroscopic spectrum Db after the Fourier transform because there is no spectroscopic interference in the specularly reflected light L1A. Therefore, the detection accuracy for various types of foreign matter F is guaranteed by combining the accumulated value of the luminance of the spectroscopic spectrum D with the Fourier transform.

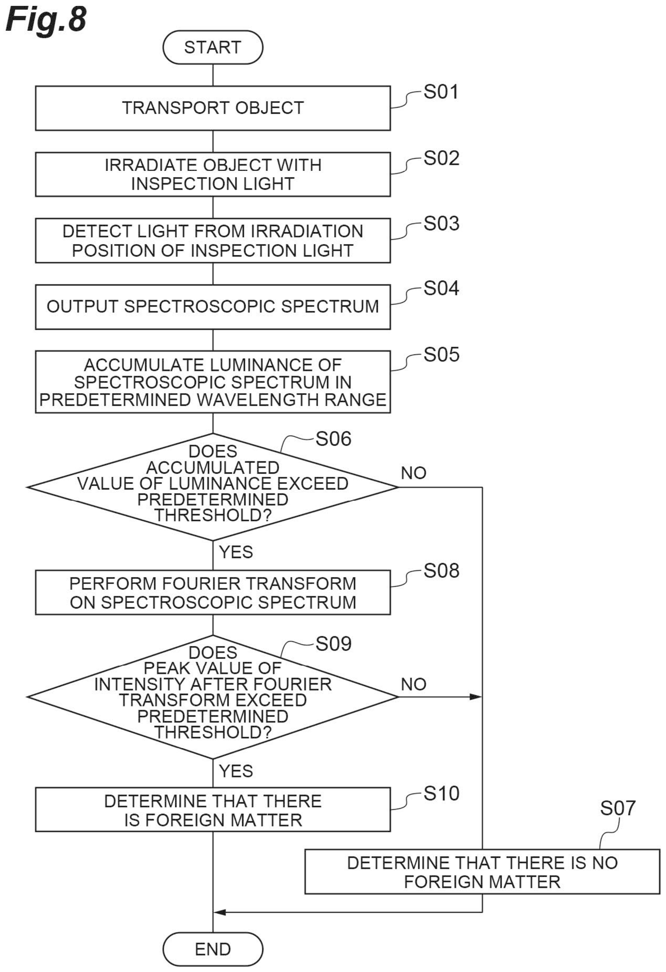

FIG. 8 is a flowchart showing an example of a foreign matter inspection method using the inspection device 1. As shown in FIG. 8, in the inspection device 1, the object S is first transported by the transport unit 2 (step S01). Moreover, the irradiation unit 3 irradiates the object S with the inspection light L0 (step S02), and the spectroscopic detection unit 4 detects the light L1 from the irradiation position R of the inspection light L0 (step S03). In the spectroscopic detection unit 4, the spectroscopic spectrum D based on the detection result of the light L1 from the irradiation position R is generated and output to the determination unit 5 (step S04).

In the determination unit 5, which has received the spectroscopic spectrum D, the luminance of the spectroscopic spectrum D is accumulated in the predetermined wavelength range (step S05). Subsequently, the determination unit 5 determines whether or not the accumulated value of the luminance of the spectroscopic spectrum D in the predetermined wavelength range exceeds the predetermined threshold (step S06). When the accumulated value of the luminance of the spectroscopic spectrum D in the predetermined wavelength range does not exceed the predetermined threshold in step S06, it is determined that there is no foreign matter F at the irradiation position R (step S07).

When the accumulated value of the luminance of the spectroscopic spectrum D in the predetermined wavelength range does not exceed the predetermined threshold in step S06, the determination unit 5 converts the spectroscopic spectrum D into an amplitude per wavenumber (a reciprocal of a wavelength) and subsequently performs the Fourier transform (step S08). Subsequently, the determination unit 5 determines whether or not the peak value of the intensity after the Fourier transform exceeds the predetermined threshold (step S09). When the peak value of the intensity after the Fourier transform does not exceed the predetermined threshold in step S09, it is determined that there is no foreign matter F at the irradiation position R (step S07). When the peak value of the intensity after the Fourier transform exceeds the predetermined threshold in step S09, it is determined that there is foreign matter F at the irradiation position R (step S10).

As described above, the inspection device 1 determines the presence or absence of the foreign matter F at the irradiation position R of the object S based on the information about the periodicity of the spectroscopic spectrum D indicating the luminance per wavelength at the irradiation position R of the inspection light L0. When the foreign matter F is the transparent substance, the inspection light L0 is incident on the foreign matter F, such that the specularly reflected light L1C specularly reflected on the surface of the foreign matter F, the specularly reflected light L1E transmitted through the foreign matter F and reflected on the back side, and the multiple-reflected light L1F in which the light transmitted through the foreign matter F is multiple-reflected within the foreign matter may be generated. Because the reflected light beams intensify or weaken each other in accordance with a phase difference determined by a wavelength and an optical path length, when the detected reflected light is spectrally separated, the spectroscopic spectrum D having the periodicity of the peaks and valleys on the wavelength axis is obtained. Therefore, by using the inspection light L0 containing a plurality of wavelengths to spectrally separate these reflected light and acquiring information about the periodicity of the spectroscopic spectrum D, it is possible to accurately detect the presence or absence of the foreign matter F, which is the transparent substance.

In the present embodiment, the determination unit 5 performs the Fourier transform on the spectroscopic spectrum D and determines the presence or absence of the foreign matter F at the irradiation position R by comparing the peak value of the intensity after the Fourier transform with the predetermined threshold. If the Fourier transform is performed on the spectroscopic spectrum D, it is possible to easily ascertain whether or not there is a periodic interference component in the spectroscopic spectrum D based on whether or not a peak appears in the intensity after the Fourier transform.

Therefore, it is possible to perform more accurate detection of the presence or absence of the foreign matter F, which is the transparent substance.

In the present embodiment, the determination unit 5 accumulates the luminance of the spectroscopic spectrum D in the predetermined wavelength range and performs the Fourier transform on the spectroscopic spectrum D when the accumulated value exceeds the predetermined threshold. When the foreign matter F, which is the transparent substance, is present, because the specularly reflected light L1C from the foreign matter F is detected, the luminance of the detected light tends to be higher than that of an area where there is no foreign matter F. Therefore, by performing the Fourier transform on the spectroscopic spectrum D only when the accumulated value of the luminance of the spectroscopic spectrum D in a predetermined wavelength range exceeds a predetermined threshold, it is possible to reduce the processing load in the determination unit 5 until the presence or absence of the foreign matter F is determined.

In the present embodiment, the wavelength range included in the inspection light L0 is greater than or equal to 1000 nm and less than 2500 nm as a shortwave infrared range. As described above, the phase difference of reflected light is determined by the wavelength of light and the optical path length. Therefore, if the thickness of the foreign matter F that is the transparent substance increases, it is considered that the periodicity of the spectroscopic spectrum D is short and the accuracy of determining the presence or absence of the foreign matter F decreases. On the other hand, by setting the wavelength contained in the inspection light L0 to a long wavelength as in the above-described range, the periodicity of the spectroscopic spectrum D is extended even if the thickness of the foreign matter F is large, and the accuracy of determining the presence or absence of the foreign matter F can be sufficiently guaranteed.

In the present embodiment, the irradiation unit 3 includes the line illuminator 11. Thereby, a plurality of irradiation positions R can be formed on the object S at once. Therefore, it is possible to quickly inspect the foreign matter of the object S.

In the present embodiment, the spectroscopic detection unit 4 includes a multi-wavelength spectral camera. By using a multi-wavelength spectral camera as the spectroscopic detection unit 4, for example, it is possible to acquire a linear spectroscopic spectrum with a resolution of 100 wavelengths or more. Therefore, the periodicity of the spectroscopic spectrum D can be acquired in a sufficient wavelength range, and the presence or absence of the foreign matter F can be suitably determined.

In the present embodiment, the inspection device 1 includes the transport unit 2 configured to transport the object S in the predetermined direction. Thereby, because the irradiation position R of the inspection light L0 can be scanned for the object S, it is possible to quickly and easily determine the presence or absence of foreign matter F over a wide range of the object S.

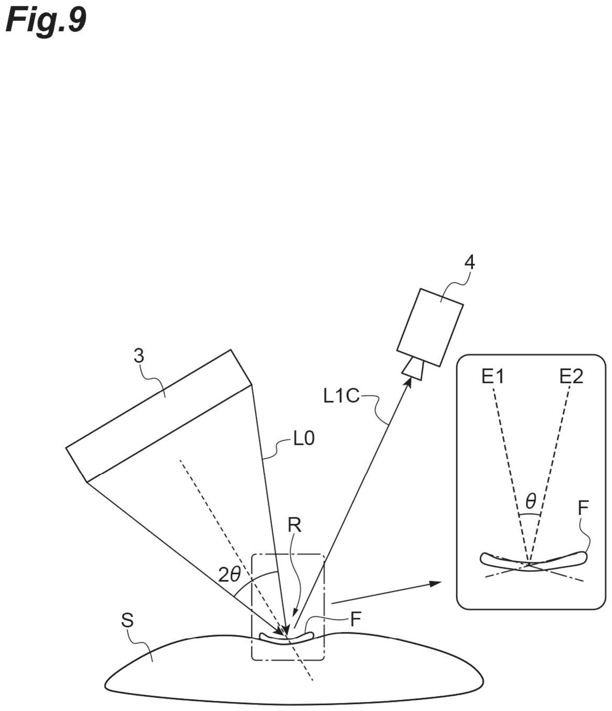

The present disclosure is not limited to the above-described embodiments. For example, although the irradiation unit 3 includes the line illuminator 11 in the above-described embodiment, the irradiation unit 3 may include a dome illuminator 21 or a flat dome illuminator 31. When there is an unevenness or slope on the surface of object S, it is considered that the inspection light L0 does not hit the surface of the object S and a shadow is formed. For example, assuming that there is an angular range θ between perpendicular components E1 and E2 relative to the surface of the foreign matter F, which forms a concave shape along the surface of the object S as shown in FIG. 9, a wide range of illumination that can cover an incidence angle 2θ is required to detect the specularly reflected light L1C corresponding to the perpendicular components E1 and E2. For such a wide range of illumination, the dome illuminator 21 or the flat dome illuminator 31 can be used instead of the line illuminator 11.

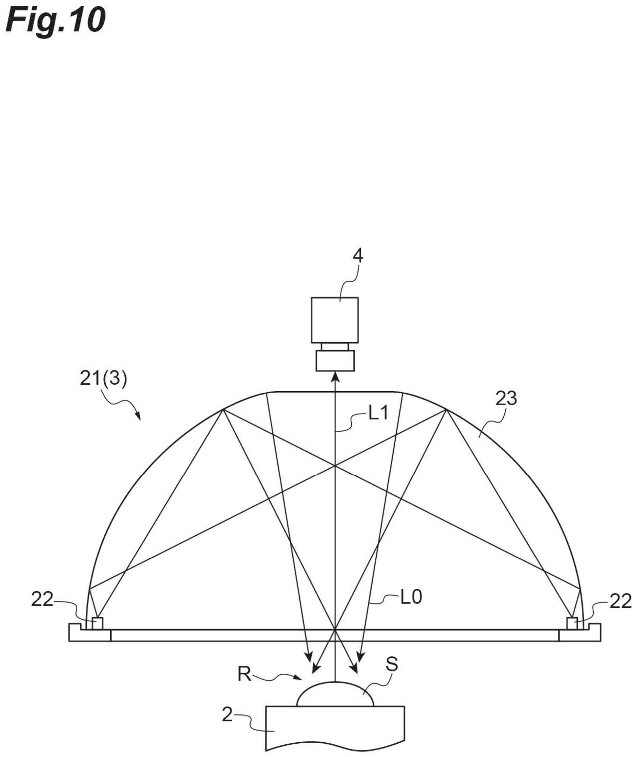

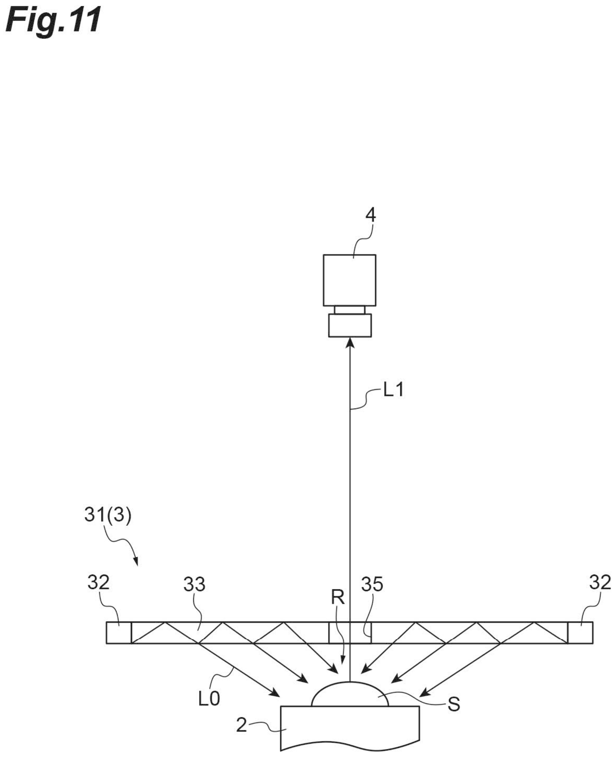

The dome illuminator 21 includes, for example, a light-emitting device 22 and a dome portion 23, as shown in FIG. 10. The inspection light L0 emitted from the light-emitting device 22 is reflected and diffused on the inner surface of the dome portion 23, and is radiated to the object S over a wide area. The flat dome illuminator 31, for example, is configured to include a light-emitting device 32 and a light guide plate 33 with an observation window 35, as shown in FIG. 11. As the light-emitting device 32, for example, an LED, a super luminescent diode (SLD), a laser light source, a halogen lamp, a fluorescent lamp, an organic EL, or the like can be used, the inspection light L0 emitted from the light-emitting device 32 is reflected and diffused in the light guide plate 33 and irradiated to the object S over a wide area. By using the dome illuminator 21 or the flat dome illuminator 31 as the irradiation unit 3, it is possible to cover an angle range corresponding to the unevenness or slope of the surface of the object S, and the accuracy of determining the presence or absence of the foreign matter F can be sufficiently guaranteed even if there is an unevenness or slope on the surface of the object S.

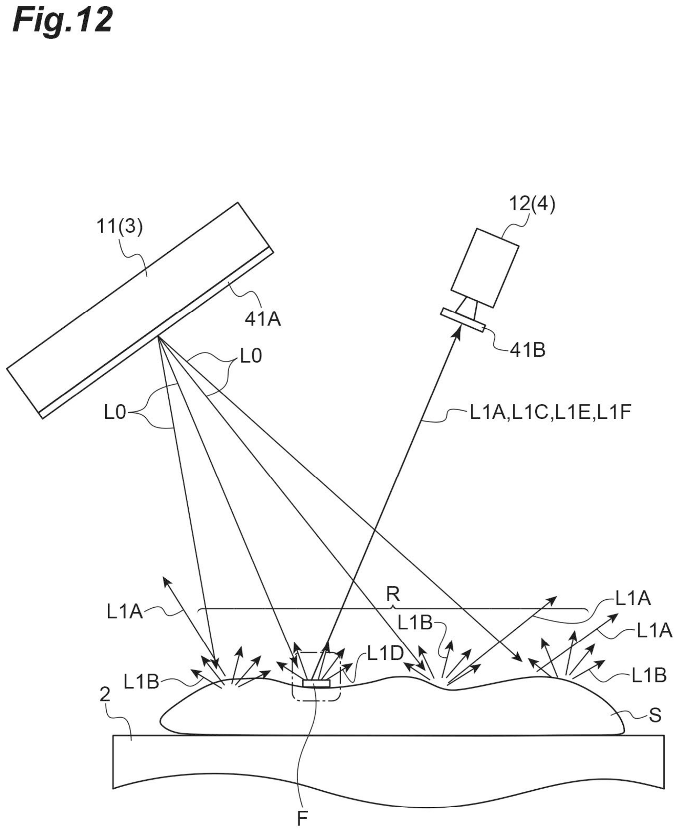

Moreover, from the viewpoint of eliminating background influences, for example, polarizers 41A and 41B, which are in a parallel Nicol relationship, may be provided in the irradiation unit 3 and the spectroscopic detection unit 4, as shown in FIG. 12. The polarizers 41A and 41B are linear polarizers with absorption axes in a predetermined direction, and are arranged on the front surface of the irradiation unit 3 and the front surface of the spectroscopic detection unit 4 so that the absorption axes are in the same direction as each other. In the specularly reflected light L1C and L1E and the multiple-reflected light L1F from the foreign matter F, the polarized state of the inspection light L0 radiated to the object S is maintained, while the diffusely reflected light L1B on the surface of the object S is non-polarized. Therefore, by providing the polarizers 41A and 41B, which are in a parallel Nicol relationship, in the irradiation unit 3 and the spectroscopic detection unit 4, background influences caused by the diffusely reflected light L1B and the like can be eliminated, and the presence or absence of the foreign matter F, which is a transparent substance, can be detected with higher accuracy.

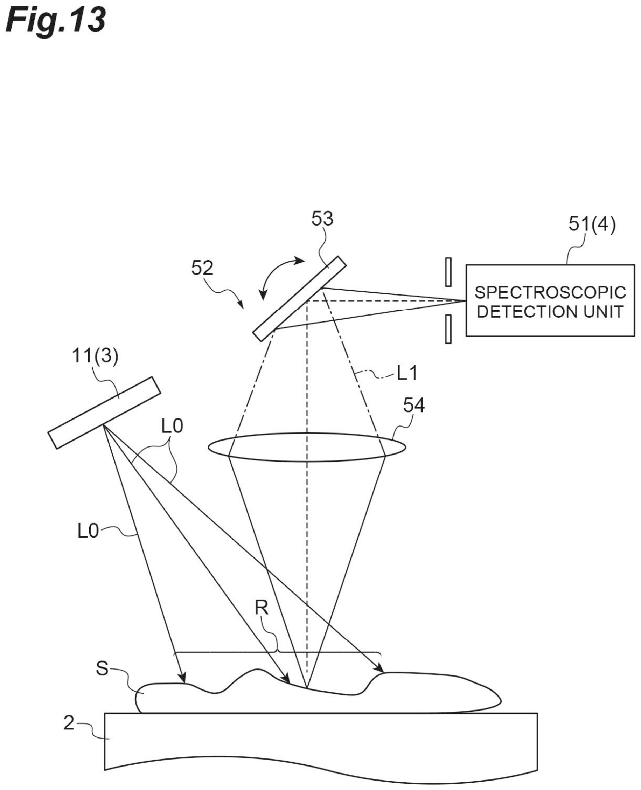

The irradiation unit 3 and the spectroscopic detection unit 4 can further take different variants. For example, as shown in FIG. 13, the irradiation unit 3 includes the line illuminator 11, the spectroscopic detection unit 4 includes a point spectroscopic detector 51, and a configuration having a scanner 52 for scanning the detection field of view of the spectroscopic detection unit 4 may be adopted. The point spectroscopic detector 51 is a camera with a built-in one- or two-dimensional image sensor and a grating, and outputs point spectroscopic spectrum data of one axis only on the wavelength axis.

The scanner 52, for example, includes a rotatable mirror 53, such as a galvanometer mirror or a MEMS mirror, and is arranged on an optical path between an imaging lens 54 and the spectroscopic detection unit 4. The scanner 52 scans the detection field of view of the spectroscopic detection unit 4 in the direction perpendicular to the transport direction of the object S (the direction of extension of the line-shaped irradiation position R formed by the line illuminator 11) according to the rotation of the mirror 53. Light L1 from the object S is condensed by the imaging lens 54, reflected by the mirror 53, and input to the spectroscopic detection unit 4.

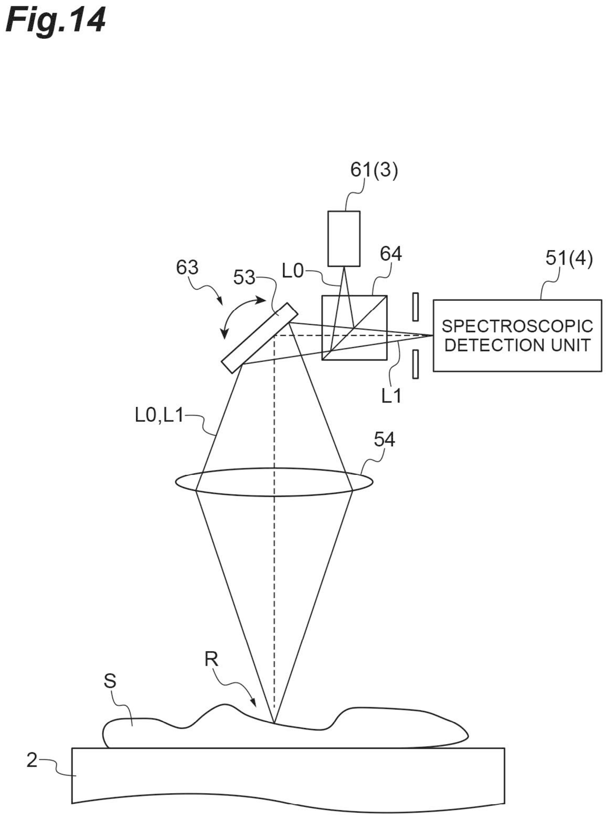

Moreover, for example, as shown in FIG. 14, the irradiation unit 3 includes a point illuminator 61, and the spectroscopic detection unit 4 includes a point spectroscopic detector 51, and a configuration with a scanner 63 for scanning the irradiation position R of the irradiation unit 3 and the detection field of view of the spectroscopic detection unit 4 may be adopted. The point illuminator 61 has a built-in single light-emitting device.

The scanner 63 includes the above-described mirror 53 and a beam splitter 64. The scanner 63 reflects the inspection light L0 emitted from the irradiation unit 3 towards the mirror 53 with the beam splitter 64, and scans the irradiation position R of the irradiation unit 3 in a direction perpendicular to the transport direction of the object S (an extension direction of the line-shaped irradiation position R formed by the line illuminator 11) according to the rotation of the mirror 53. Light L1 from the object S is condensed by the imaging lens 54, reflected again by the mirror 53, de-scanned, and input to the spectroscopic detection unit 4 after being transmitted through the beam splitter 64.

According to the configurations shown in FIGS. 13 and 14, the spectroscopic detection unit 4 can be constructed using an existing spectroscopic detector without using a high-performance camera such as a multispectral camera or a hyperspectral camera. Therefore, it is possible to construct the inspection device 1 at a low cost.

In the present embodiment, the presence or absence of foreign matter F, which is a transparent substance, can be accurately detected by acquiring information about the periodicity of the spectroscopic spectrum D, but foreign matter information such as the thickness and type of the foreign matter F may be further analyzed based on the spectroscopic spectrum D if it is determined that there is foreign matter F.

Reference Signs List

1 Inspection device, 2 Transport unit, 3 Irradiation unit, 4 Spectroscopic detection unit, 5 Determination unit, 11 Line illuminator, 21 Dome illuminator, 31 Flat dome illuminator, 41A, 41B Polarizer, 51 Point spectroscopic detector, 52, 63 Scanner, S Object, F Foreign matter, R Irradiation position, L0 Inspection light, L1 Light from irradiation position, D Spectroscopic spectrum

**Claims**

1. An inspection device configured to detect foreign matter, which is a transparent substance, in an object, the inspection device comprising:

    an irradiation unit configured to irradiate the object with inspection light including a plurality of wavelengths;
    a spectroscopic detection unit configured to detect light from an irradiation position of the inspection light on the object and output a spectroscopic spectrum indicating luminance of each wavelength at the irradiation position; and
    a determination unit configured to determine presence or absence of the foreign matter at the irradiation position based on information regarding periodicity of the spectroscopic spectrum.

2. The inspection device according to claim 1, wherein the determination unit configured to perform a Fourier transform on the spectroscopic spectrum and determine the presence or absence of the foreign matter at the irradiation position by comparing a peak value of an intensity after the Fourier transform with a predetermined threshold.

3. The inspection device according to claim 2, wherein the determination unit configured to accumulate the luminance of the spectroscopic spectrum in a predetermined wavelength range and perform the Fourier transform on the spectroscopic spectrum when an accumulated value exceeds a predetermined threshold.

4. The inspection device according to any one of claims 1 to 3, wherein a wavelength range included in the inspection light is greater than or equal to 1000 nm and less than 2500 nm.

5. The inspection device according to any one of claims 1 to 4, wherein the irradiation unit includes a line illuminator.

6. The inspection device according to any one of claims 1 to 5, wherein the irradiation unit includes a dome illuminator or a flat dome illuminator.

7. The inspection device according to any one of claims 1 to 6, wherein the spectroscopic detection unit includes a multi-wavelength spectral camera.

8. The inspection device according to any one of claims 1 to 4,

    wherein the irradiation unit includes a line illuminator,
    wherein the spectroscopic detection unit includes a point spectroscopic detector, and
    wherein the inspection device has a scanner configured to scan a detection field of view of the spectroscopic detection unit.

9. The inspection device according to any one of claims 1 to 4,
wherein the irradiation unit includes point illumination,

    wherein the spectroscopic detection unit includes a point spectroscopic detector, and
    wherein the inspection device has a scanner configured to scan an irradiation position of the irradiation unit and a detection field of view of the spectroscopic detection unit.

10. The inspection device according to any one of claims 1 to 9, wherein a polarizer in a parallel Nicol relationship is provided in each of the irradiation unit and the spectroscopic detection unit.

11. The inspection device according to any of claims 1 to 10, further comprising a transport unit configured to transport the object in a predetermined direction.

**Fig.1**

*Fig.2*

(a)

(b)

WAVELENGTH

(c)

WAVELENGTH

# *Fig.3*

# Fig.4

# Fig.5

(a)

LUMINANCE

WAVELENGTH

D

(b)

AMPLITUDE

WAVENUMBER

Da

(c)

INTENSITY

FREQUENCY

Db

*Fig.6*

(a)

POLYETHYLENE FRAGMENT : THICKNESS 10 μm

WAVELENGTH nm

(b)

POLYETHYLENE FRAGMENT : THICKNESS 50 μm

WAVELENGTH nm

(c)

POLYETHYLENE FRAGMENT : THICKNESS 100 μm

WAVELENGTH nm

**Fig.7**

(a)

POLYETHYLENE FRAGMENT : THICKNESS 10 μm

WAVELENGTH nm

(b)

POLYETHYLENE FRAGMENT : THICKNESS 50 μm

WAVELENGTH nm

(c)

POLYETHYLENE FRAGMENT : THICKNESS 100 μm

WAVELENGTH nm

## Fig.8

```
                    START

S01  TRANSPORT OBJECT

S02  IRRADIATE OBJECT WITH
     INSPECTION LIGHT

S03  DETECT LIGHT FROM IRRADIATION
     POSITION OF INSPECTION LIGHT

S04  OUTPUT SPECTROSCOPIC SPECTRUM

S05  ACCUMULATE LUMINANCE OF
     SPECTROSCOPIC SPECTRUM IN
     PREDETERMINED WAVELENGTH RANGE

S06  DOES ACCUMULATED VALUE OF
     LUMINANCE EXCEED PREDETERMINED
     THRESHOLD?                          NO

     YES

S08  PERFORM FOURIER TRANSFORM
     ON SPECTROSCOPIC SPECTRUM

S09  DOES PEAK VALUE OF INTENSITY
     AFTER FOURIER TRANSFORM EXCEED
     PREDETERMINED THRESHOLD?            NO

     YES

S10  DETERMINE THAT THERE
     IS FOREIGN MATTER                   S07  DETERMINE THAT THERE IS NO
                                              FOREIGN MATTER

                    END
```

# Fig.9

# Fig.10

# *Fig.11*

# *Fig.12*

# Fig.13

SPECTROSCOPIC DETECTION UNIT

# Fig.14

## INTERNATIONAL SEARCH REPORT

| International application No. |
| --- |
| **PCT/JP2024/022888** |

| | |
| --- | --- |
| **A.** | **CLASSIFICATION OF SUBJECT MATTER** |
| | ***G01N 21/89***(2006.01)i |
| | FI:   G01N21/89 Z; G01N21/89 H |

According to International Patent Classification (IPC) or to both national classification and IPC

| | |
| --- | --- |
| **B.** | **FIELDS SEARCHED** |

Minimum documentation searched (classification system followed by classification symbols)

　　G01N21/00-21/958; G01J3/00-4/04;

Documentation searched other than minimum documentation to the extent that such documents are included in the fields searched

　　Published examined utility model applications of Japan 1922-1996
　　Published unexamined utility model applications of Japan 1971-2024
　　Registered utility model specifications of Japan 1996-2024
　　Published registered utility model applications of Japan 1994-2024

Electronic data base consulted during the international search (name of data base and, where practicable, search terms used)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X | JP 2020-204579 A (SEDI INC.) 24 December 2020 (2020-12-24) | 1, 4 |
| | paragraphs [0012]-[0015], [0025]-[0042], fig. 1, 5-8 | |
| Y | paragraphs [0012]-[0015], [0025]-[0042], fig. 1, 5-8 | 5-11 |
| A | paragraphs [0012]-[0015], [0025]-[0042], fig. 1, 5-8 | 2-3 |
| Y | JP 2016-540996 A (TOMRA SORTING NV) 28 December 2016 (2016-12-28) | 5, 7-11 |
| | paragraphs [0015], [0040], [0046], [0056], [0058], [0060], fig. 1 | |
| Y | JP 2018-4509 A (RICOH COMPANY, LTD.) 11 January 2018 (2018-01-11) | 6, 10 |
| | paragraph [0046], fig. 12 | |
| Y | JP 2009-257903 A (HITACHI HIGH-TECHNOLOGIES CORPORATION) 05 November 2009 (2009-11-05) | 10 |
| | paragraphs [0001], [0033] | |
| A | CN 108645793 A (WUHAN CHINA STAR OPTOELECTRONICS SEMICONDUCTOR DISPLAY TECHNOLOGY CO., LTD.) 12 October 2018 (2018-10-12) | 1-11 |

| ☐ Further documents are listed in the continuation of Box C. | ☑ See patent family annex. |
| --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **16 July 2024** | **30 July 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

International application No.

**PCT/JP2024/022888**

| Patent document cited in search report | | | Publication date (day/month/year) | Patent family member(s) | | Publication date (day/month/year) |
|---|---|---|---|---|---|---|
| JP | 2020-204579 | A | 24 December 2020 | US 2020/0400587 A1 paragraphs [0027]-[0031], [0041]-[0058], fig. 1, 5-8B CN 112103200 A TW 202113347 A | | |
| JP | 2016-540996 | A | 28 December 2016 | US 2016/0252461 A1 paragraphs [0014], [0040], [0046], [0064], [0066], [0068], fig. 1a WO 2015/063300 A1 CA 2928878 A1 CN 105874321 A | | |
| JP | 2018-4509 | A | 11 January 2018 | (Family: none) | | |
| JP | 2009-257903 | A | 05 November 2009 | US 2009/0279079 A1 paragraphs [0001], [0068] US 2012/0236296 A1 | | |
| CN | 108645793 | A | 12 October 2018 | (Family: none) | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021189071 A